# EUROPEAN PATENT SPECIFICATION

(11) **EP 1 174 513 B1**
(45) Date of publication and mention of the grant of the patent: **21.02.2007**
(21) Application number: 00870230.0
(22) Date of filing: 12.10.2000
(51) Int. Cl.: C12N 15/82, A01H 5/00

(54) **A method for obtaining a genetically modified plant**
Verfahren zur Herstellung einer genetisch-verändereten Pflanze
Procédé d'obtention d'une plante génétiquement modifiée

(30) Priority: 17.07.2000 US 617659
(43) Date of publication of application: 23.01.2002
(73) Proprietor: Ses Europe N.V./S.A., 3300 Tienen (BE)
(72) Inventor: Hall, Robert David, 6706 EA Wageningen (NL); Bruinsma, Tjitske Riksen, 6871 ZJ Renkum (NL); Weyens, Guy, B- 1650 Beersel (BE); Lathouwers, Jean, B- 1830 Machelen (BE); Lefebvre, Marc, B- 1370 Jodoigne (Souveraine) (BE)
(74) Representative: Van Malderen, Joëlle

(56) References cited:
- WO-A-01/05936
- WO-A-95/10178
- FU X ET AL: "LINEAR TRANSGENE CONSTRUCTS LACKING VECTOR BACKBONE SEQUENCES GENERATE LOW-COPY-NUMBER TRANSGENIC PLANTS WITH SIMPLE INTEGRATION PATTERNS" TRANSGENIC RESEARCH, LONDON, GB, no. 9, 2000, pages 11-19, XP002908639 ISSN: 0962-8819
- HOLM P B ET AL: "TRANSFORMATION OF BARLEY BY MICROINJECTION INTO ISOLATED ZYGOTE PROTOPLASTS" TRANSGENIC RESEARCH, LONDON, GB, vol. 9, February 2000 (2000-02), pages 21-32, XP000945396 ISSN: 0962-8819

### SEQUENCE LISTING

<110> SES Europe N.V./S.A.
<120> A METHOD FOR OBTAINING A GENETICALLY MODIFIED PLANT
<130> P.SES.05/EP
<140> 00870230.0
   <141> 2000-10-12
<160> 8
<170> Patent In Ver. 2.1
<210> 1
   <211> 20
   <212> DNA
   <213> Artificial Sequence
<220>
   <223> Description of Artificial Sequence: primer
<400> 1
   ggctcgtatg ttgtgtggaa 20
<210> 2
   <211> 20
   <212> DNA
   <213> Artificial Sequence
<220>
   <223> Description of Artificial Sequence: primer
<400> 2
   gagtcagtga gcgaggaagc 20
<210> 3
   <211> 20
   <212> DNA
   <213> Artificial Sequence
<220>
   <223> Description of Artificial Sequence: primer
<400> 3
   ctgcaaggcg attaagttgg 20
<210> 4
   <211> 20
   <212> DNA
   <213> Artificial Sequence
<220>
   <223> Description of Artificial Sequence: primer
<400> 4
   gcacagatgc gtaaggagaa 20
<210> 5
   <211> 20
   <212> DNA
   <213> Artificial Sequence
<220>
   <223> Description of Artificial Sequence: primer
<400> 5
   gcgaaaccct ataagaaccc 20
<210> 6
   <211> 20
   <212> DNA
   <213> Artificial Sequence
<220>
   <223> Description of Artificial Sequence: primer
<400> 6
   agctcggtac ccactggatt 20
<210> 7
   <211> 25
   <212> DNA
   <213> Artificial Sequence
<220>
   <223> Description of Artificial Sequence: border sequence
<400> 7
   ggtttacccg ccaatatatc ctgtc 25
<210> 8
   <211> 24
   <212> DNA
   <213> Artificial Sequence
<220>
   <223> Description of Artificial Sequence: border sequence
<400> 8
   atttacaatt gaatatatcc tgcc 24

## Description

### Field of the invention

This invention is related to a method for obtaining a genetically modified plant. More particularly, the invention is related to a method for the genetic transformation of plant cells, the regeneration of whole plants from such transformed plant cells.

Particularly, but not exclusively, the invention is related to a method for obtaining genetically modified plant of Beta vulgaris type.

This invention is related to new genetically modified plants, preferably obtained by said method.

### Background of the invention

Plants are susceptible to different diseases caused by organisms such as viruses, parasites, insects, nematodes or by chemical products. This susceptibility has great economical consequences with estimated losses of several billions of dollars each year.

Alternatively, but also jointly to the use of chemical products such as herbicides and insecticides, plant genetic transformation techniques have been proposed to overcome this problem. The final goal of these techniques is to obtain genetically transformed plants. For this purpose, the gene of interest has to be transferred into plant cells. Stable transformed plant cells, i.e. plant cells having integrated said gene of interest into their genome, have then to be selected and have to be used for regeneration of whole plants.

Two parameters are critical for the success of genetic transformation techniques: the transformation frequency of plant cells which has to be high and the capacity of transformed cells to regenerate whole plants.

The transformation techniques can be classified into physical DNA delivery methods such as electroporation, PEG-mediated DNA uptake, biolistics and methods based on Agrobacterium-mediated DNA transfer.

However, all the current techniques have as common disadvantage the use procaryotic or plasmidic nucleotidic sequences associated with the gene of interest for the selection of transformed plant cells.

For example, the international patent application WO 95/10178 describes a method for the genetic transformation of a plant by introducing a DNA construct including a selectable marker gene into a stomatal cell of the plant, said stomatal cell being converted to a protoplast prior to regeneration.

The transformation is conducted on a cell population containing guard cells. However, said complex method presents several drawbacks, because the hereditary material is a plasmid DNA produced in E. coli, purified and used in its entirety. For a direct DNA transfer method, any region or all of the plasmid DNA may become stably integrated into the plant genome. This means that the selectable marker and target transgenes which are part or all of the bacterial plasmid sequence (containing bacterial antibiotic selectable markers such as nptIII) may be cointegrated or even separately integrated in a different genomic site. In addition, the borders of the gene of interest to be integrated into the genome of the plant can be submitted to the action of exonucleases.

The integration of such procaryotic or plasmidic nucleotidic sequences into the plant genome can lead to plant disorders. Therefore, it exists a need for genetically modified plants that do not incorporate selectable markers or other genes issued from heterologous species, except the genetic sequence of interest.
Fu et al (2000, Transgenic Research 9:11-19) disclose rice tissue bombardment with a linear DNA fragment isolated from a plasmid, corresponding to a minimal bar gene expression cassette (promoter, open reading frame and terminator). Transformation with such minimal constructs resulted in predominantly 'simple' integration events and a low frequency of transgene rearrangements. Gene expression was obtained, however, some drawbacks remain when using this method.
Holm et al (2000, Transgenic Research 9:21-32) disclose the transformation of barley by microinjection into isolated zygote protoplasts. Gene expression was rarely achieved.

### Aims of the invention

The present invention aims to provide a method for genetic transformation of plant cells with a genetic sequence of interest and for regeneration of whole plants from transformed cells thus obtained, which is simple to be used and which does not present the drawbacks of the state of the art.

The present invention aims to provide such a method for genetic transformation of plant cells using techniques which result in a genetically modified plant which is free or substantially free from prokaryotic or plasmidic nucleotide sequences (especially selectable markers which allow an antibiotic resistance).

### Summary of the invention

The present invention is related to a method for obtaining a genetically modified plant comprising the steps of
(i) transforming protoplasts, preferably derived from a conversion of stomatal cells, by introducing an hereditary material into the protoplasts of said plant in order to obtain transformants containing at least one copy of the hereditary material per protoplast,
(ii) selecting a stable transformant and
(iii) regenerating plant cells or a whole plant from said transformant,
said method being characterised in that the hereditary material is a cassette nucleotide sequence to be inserted in the genome of said plant and being free or substantially free from other prokaryotic or plasmidic nucleotide sequences, especially selectable markers encoding an antibiotic resistance.

It is meant by "cassette nucleotide sequence" a sequence consisting of only a nucleotide sequence of interest to be inserted in the genome of said plant, associated with one or more promotor(s) and terminal nucleotide sequence(s) and possibly regulatory sequences sufficient to obtain an efficient expression of said sequence of interest into the plant.

Preferably, said cassette nucleotide sequence is obtained by classical amplification techniques using nucleotide sequences as templates to start off genetic amplification.

Advantageously, said amplification technique is selected from the group consisting of PCR, RTPCR, LCR, CPT, NASBA, ICR or avalanche DNA amplification techniques well known by the person skilled in the art.

Advantageously, said templates are deleted before the transformation of said protoplasts by using specific known genetic tools such as nuclease or restriction enzymes and the resulting nucleotide cassette is free or essentially free from other nucleotide sequences (from bacterial or from other origin) than the sequences of interest.

It is meant by the genome of a plant being free or substantially free from other prokaryotic or plasmidic nucleotide sequences, especially selectable markers encoding antibiotic resistance, the fact that said genome of the plant does not comprise a regulatory or encoding nucleotide sequence of heterologous origin than the cassette sequence to be inserted into the genome of the plant and except few portions of nucleotide sequences of prokaryotes which have not been completely deleted from the cassette nucleotide sequence after amplification.

Said portions of nucleotide sequences comprise less than 50 nucleotides, preferably less than 20 nucleotides, more preferably less than 10 nucleotides, which do not comprise prokaryotic polypeptides or do not comprise promoter/operated sequences of prokaryotic origin.

The method according to the present invention preferably comprises also (as preliminary steps) the steps of providing an intact tissue of said plant containing stomatal cells and converting said stomatal cells into protoplasts by enzymatic digestion of the cell walls, said steps being performed before the step of transforming said protoplasts, said plant tissue being preferably senescent leaf tissue or leaf epidermis.

Preferably, the stomatal cells correspond to a cell population containing guard cells or to a cell population enriched in guard cells and the step of transforming said protoplasts is advantageously conducted on a suspension of protoplasts.

Preferably, the step of transforming said protoplasts corresponds to the known PEG-mediated genetic transformation technique (Krens et al., Nature 296, 72-74 (1982)) and comprises mixing said suspension of protoplasts and the hereditary material, preferably an amplicon at a concentration of higher than 1µg preferably between about 10 and about 100µg DNA /1x 10⁶ protoplasts, as described in the example 10 of the document WO 95/10178 incorporated herein by reference.

Preferably, the plant is Beta vulgaris which is known to be a species transformable at very low frequency. According to the invention, the term Beta vulgaris includes sugar beet, fodder beet, table beet and Swiss chard.

The nucleotide sequence of interest being inserted into the plant or plant cell is preferably selected from the group consisting of genes involved into a resistance to plant viruses (especially BNYVV resistance in sugar beet), resistance to plant parasites (nematodes, fungi or insects), resistance to herbicides (preferably glyphosate, imidazolinone such as glufosinate and/or acetochloride resistance), insecticides or fungicides, or involved in the metabolism of carbohydrates (improved production of carbohydrates, polysaccharides, oligosaccharides or monosaccharides such as starch, fructans, glucose, fructose,...).

Another aspect of the present invention is related to a plant or a plant cell, preferably a sugar beet (Beta vulgaris plant or plant cell) comprising into their genome an hereditary material consisting only of a cassette nucleotide sequence, said cassette nucleotide sequence having a nucleotide sequence of interest to be inserted into the plant associated with one or more promoters and terminal nucleotide sequences and possibly regulatory sequence(s) sufficient to obtain an efficient expression of said sequence of interest into the plant; said plant or plant cell being free or substantially free from other heterologous prokaryotic or plasmidic nucleotide sequences, especially selectable markers encoding antibiotic resistance.

Advantageously, said plant or plant cell, preferably a sugar beet (Beta vulgaris plant or plant cell) is obtained by the method according to the invention.

A last aspect of the invention concerns a transgenic plant tissues of said plant, which are preferably selected from the group consisting of fruits, stems, roots, tubers or seeds.

The invention will be described in details in the following non limited example in reference to the enclosed figure.

### Short description of the drawings

- Fig. 1: represents schematically the construction of a gene cassette used in the method according to the invention and the location and direction of the primer for the amplification of said gene cassette.
- Fig. 2: represents a map of pS63.

### Detailed description of the invention

### Guard cell protoplast isolation

The sugar beet breeding line Bv-NF derived from a breeding program at Plant Research International, The Netherlands was used.

A previously developed and fully optimized protocol (described in the document WO 95/10178) for the isolation and purification of guard cell protoplasts was used to obtain large numbers of >90 % pure guard cell protoplasts. For each isolation, 1.5 g de-ribbed leaf material was harvested from 4 week old sugar beet shoot cultures. This was blended in a Waring blender at maximum speed (23000 rpm) for about 60 sec in a 250 ml metal beaker containing 50 ml sterile tap water (4°C). The clean epidermal fragments were collected on a 297µm nylon mesh ( Nybolt, Zurich, Switzerland) and washed with 200ml sterile tap water before transferring to a 9 cm Petri dish containing 10 ml preincubation medium (Krens et al., 1990) for 1 h. To recover the epidermis fraction, the suspension was centrifuged for 1 min at 55 x *g* and the supernatant discarded. Cell wall digestion took place overnight in a total volume of 50 ml CPW9M medium supplemented with 0.5% (w/v) Cellulase RS and 3% (w/v) Macerozyme R10 (both Yakult Honsha, Tokyo, Japan), pH 5.8 in 10 6 cm Petri dishes (5 ml/dish). The digestion took place in the dark at 25°C with gentle agitation.

After about 16 h, many protoplasts were generally seen floating near the surface of the medium. After gentle agitation of the suspension using a sterile pipette to release additional protoplasts still adhering to cuticle fragments, the digests were pooled and passed through 297 and 55µm nylon filters (Nybolt, Zurich, Switzerland). The filtrate was mixed with an equal volume of iso-osmotic Percoll containing 15% (w/v) sucrose (Percoll15S) and divided over 12 x 12 ml centrifuge tubes (Greiner, TC quality). In each tube, first 1ml CPW15S (Krens et al., 1990, *to complete*) and then 0.5 ml 9% (w/v) mannitol containing 1 mM CaCl₂ (9M) was carefully layered on top of the protoplast suspension. After centrifugation at 55 x *g* for 10 min (MSE Centaur 2, Fisons UK, swing out rotor), the viable guard cells were collected with the 9M layer. To concentrate the protoplasts, these layers were pooled and mixed with Percoll15S to give a final volume of 16 ml. This was then divided between two centrifuge tubes, layered again as above and recentrifuged. Careful removal of the 9M layers yielded the enriched guard cell fraction, which were routinely 70 - 95% pure. Guard cell protoplast yields are routinely 1-3 x 10⁶ /g leaf.

### Transformation, protoplast culture and selection

Transformations were performed in 12 ml centrifuge tubes, each containing about 1 x 10⁶ protoplasts suspended in 0.75 ml 9M medium. PCR DNA or plasmid DNA (50µg) was added and immediately after mixing, 0.75 ml PEG medium (40% PEG 6000 dissolved in F medium (Krens et al.,1982, *to complete*) was added dropwise. After gentle but thorough mixing, the suspension was incubated at room temperature for 30 min with intermittant agitation. Subsequently, to remove the PEG, at 5 min intervals, 4 x 2 ml aliquots of F medium were added. After centrifugation for about 5 min at 55 x *g* the supernatant was discarded and the protoplast pellet resuspended in 9M and recentrifuged. The cells were finally resuspended in 1 ml 9M for counting. Protoplasts were embedded in Ca alginate (62500 / ml) and cultured in modified, liquid K8P medium (Hall et al., 1993, *to complete*) at 28°C in the dark. To select for stably transformed cells, bialaphos was added after 7 days, to give a final concentration of 200µg/l. On day 18, the alginate discs were cut into 3 mm strips and transferred to PG1B medium (de Greef and Jacobs, 1979, containing 2 mg/l glycine and 1µM BAP) supplemented with 250µg/l bialaphos and solidified with 0.9% agarose (Seaplaque, Duchefa, Haarlem, the Netherlands). Two weeks later the resistant calli had grown out from the alginate to form colonies ± 1 mm in diameter. These were transferred to fresh selection plates and cultured for an additional 2 weeks. Calli were subsequently subcultured on nonselective De Greef and Jacobs medium supplemented with 2 mg/l glycine, 1 mg/l BAP and 2 mg/l NAA (PNB) and solidified with 0.9% agar (Daichin, Brunschwig chemie, Amsterdam, the Netherlands) and were transferred to the light (3000 lux, Philips TLD fluorescent, 16 h/8 h darkness) at 25°C. Cultures were transferred every 14 days and regeneration occurred via somatic embryogenesis within 4 weeks.

Somatic embryos were transferred to De Greef and Jacobs medium supplemented with 0.25 mg/l BAP and 0.5% S1000 agar (B&V. Gattatico, Italy) on which they developed into plants for rooting and transfer to soil.

### PCR DNA preparation

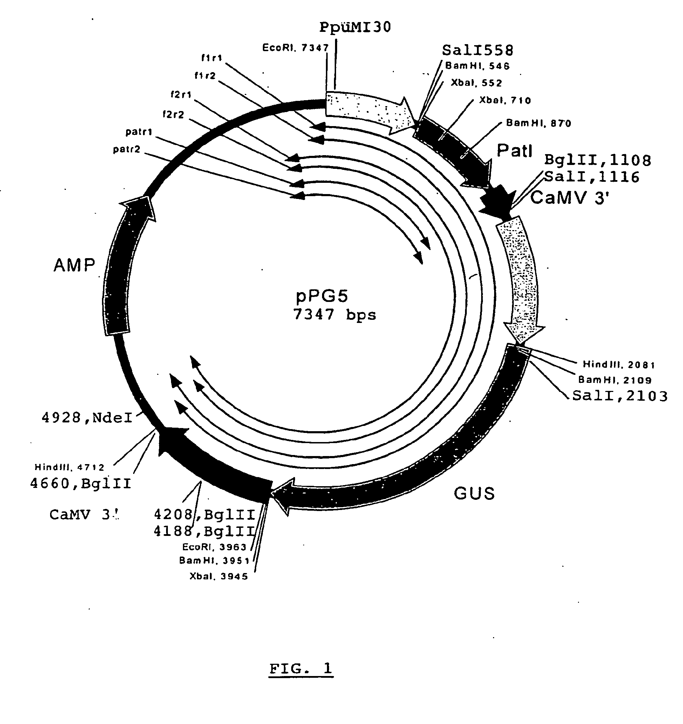

Initial transformations were performed using control plasmid DNA produced by the standard method and DNA produced via PCR. The PCR fragments used for the protoplast transformations were prepared with standard PCR techniques using a range of possible PCR primers. Four different primers (2 forward and 2 reverse) were designed for the plasmid pPG 5 ( 7347 bp) to make the 4 possible fragments all of which contained the whole gene cassette for both PAT and GUS genes irrespective of F/R primer combination. The first forward primer corresponds to a sequence close to the start of the 35S promoter of the PAT gene (f1), starting at position 7277 bp with the sequence 5'- GGCTCGTATGTTGTGTGGAA. The second forward primer starts an extra 200bp before the promoter (f2) at position 7092 bp and has the sequence 5'-GAGTCAGTGAGCGAGGAAGC. The first reverse primer corresponds to a sequence adjacent to the terminator of the GUS gene (r1) and starts at position 4788 bp and has the sequence 5'-CTGCAAGGCGATTAAGTTGG. The second reverse primer has an extra 200 bp on the terminator side (r2), and starts at position 4913 bp with the sequence 5'-GCACAGATGCGTAAGGAGAA. The locations and directions of the primers are shown in Fig 1.With these primers four fragments could be made namely: f1/r1, f1/r2, f2/r1 and f2/r2.

Reactions were performed using 35 cycles of 1 min denaturation at 94°C, 1 min annealing at 60°C, 5 min extension at 72°C with a final extra extension period of 5 min.

After the PCR reaction the DNA was concentrated by precipitation and redissolving in a small volume of sterile water. The DNA concentration was determined by measuring the OD 260nm and the fragment size was checked by gel electrophoresis. All four DNA fragments were then used separately for protoplast transformations.

In addition, two smaller PCR fragments containing only the *PAT* gene cassette (without the GUS cassette) were made using the primer f2 and one of two reverse primers, r3 or r4 both of which are close to the end of the CaMV terminator of the *PAT* gene (Fig 1). Primer r3 starts at position 1232 bp with the sequence 5'-GCGAAACCCTATAAGAACCC; primer r4 starts at position 1340 bp with the sequence 5'-AGCTCGGTACCCACTGGATT. PCR reactions were performed using 35 cycles of 1 min denaturation at 94°C, 1 min annealing at 65°C, 2 min extension at 72°C with an extension period of 5 min. Further preparation of the DNA was as described above.

Results to date are listed in Tables 2c through 7 for transgenic calli and these results suggest that transgenic calli are produced using PCR DNA from every primer combination tested. GUS positive and thus stably transformed transgenic calli have also been detected in those transformations performed using PCR DNA which also contained the GUS gene. The first plants have already been produced. These contain the transgenes. The data is listed in Table 8. The probes in the data list the number of bands in the Southern plot. The frequencies of transformation when using PCR DNA are not significantly different than those for transformations performed using the standard plasmid DNA isolation. The presence of border sequences (the extra +/- 200 bp sequence before and / or after the promoter / terminator) which were considered to perhaps be necessary to avoid potential gene inactivation through exonuclease activity do not appear to influence the transformation frequency and are therefore not required.

### Table 1

Amount of DNA molecules used in protoplasts transformation with PEG-medium :

| Fragment | Length in bp | 34µg fragment/ 1 10⁶ protoplasts | 50µg fragment/ 1 10⁶ protoplasts |
|---|---|---|---|
| Ppg5 | 7347 | | 6.4 10¹² |
| R1/f2 | 4983 | 6.4 10¹² | 9.5 10¹² |
| R1/f3 | 5170 | | 9.2 10¹² |
| R4/f2 | 4858 | | 9.7 10¹² |
| R4/f3 | 5043 | | 9.4 10¹² |
| Patr1/f2 | 1410 | | 33.6 10¹² |
| Patr2/f2 | 1302 | | 36.4 10¹² |

**Table 2a**

| No. of experiment | Fragment | Amount of protoplas ts used | Amount of callus | Amount of plants |
|---|---|---|---|---|
| f1 | ppg5 | 368.000 | 12 | |
| | r1/f2 34µg | 474.000 | 9 | |
| | | 513.000 | 20 | 1 |
| | 50µg | | | |
| f2 | Besmet (contaminated) | | | |
| f3 | Besmet (contaminated) | | | |
| f4 | ppg5 | 710.000 | 34 | |
| | r1/f2 34µg | 702.000 | 39 | 2 |
| | | 627.000 | 47 | 2 |
| | 50µg | | | |
| f5 | ppg5 | 548.000 | 5 | |
| | r1/f3 | 2.100.000 | 16 | 1 |
| f6 | ppg5 | 513.000 | 4 | |
| | r4/f2 | 1.700.000 | 46 | 2 |
| | r4/f3 | 1.000.000 | 20 | |
| f7 | ppg5 | 1.000.000 | 12 | |
| | r1/f3 | 734.000 | 19 | |
| | r4/f2 | 913.000 | 28 | |
| | r4/f3 | 792.000 | 10 | |
| f8 | r1/f2 | 648.000 | 70 | |
| | r1/f3 | 616.000 | 61 | |
| | r4/f2 | 598.000 | 30 | |
| | r4/f3 | 636.000 | 7 | |
| | patr1 | 460.000 | 61 | |
| | patr2 | 427.000 | 48 | |
| f9 | ppg5 | 508.000 | 46 | |
| | patr1 | 1.200.000 | 130 | |
| | patr2 | 1.400.000 | 40 | |
| f10 | ppg5 | 266.000 | | |
| | patr1 | 464.000 | | |
| | Patr2 | 852.000 | | |
| | ppg5 | 4.605.000 | 113 | |
| | r1/f2 | 2.964.000 | 185 | 5 |
| | r1/f3 | 3.450.000 | 96 | 1 |
| | r4/f2 | 3.211.000 | 104 | 2 |
| | r4/f3 | 2.428.000 | 37 | |
| | patr1 | 2.124.000 | 191 | |
| | patr2 | 2.679.000 | 88 | |

**Table 2b: Summary of Table 2a**

| Fragment | Amount of protoplasts used | Amount of callus | Amount of plants |
|---|---|---|---|
| ppg5 | 4.605.000 | 129 | 1 |
| r1/f2 | 2.964.000 | 185 | 4 |
| r1/f3 | 3.450.000 | 96 | 1 |
| r4/f2 | 3.211.000 | 104 | 7 |
| r4/f3 | 2.428.000 | 37 | |
| patr1 | 2.124.000 | 222 | 4 |
| patr2 | 2.679.000 | 100 | |

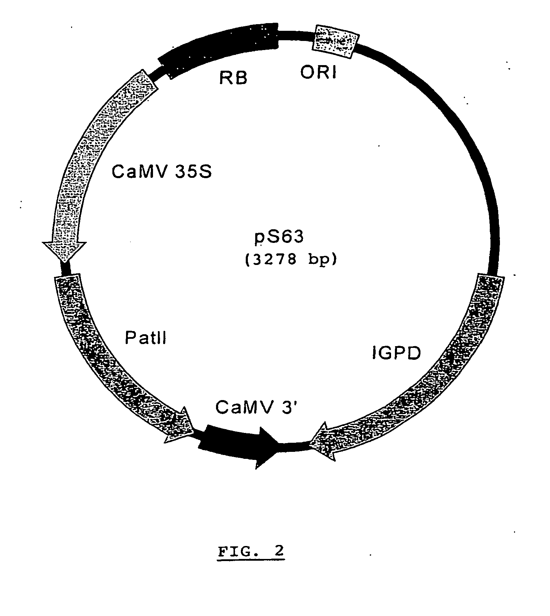

Fig. 2 is the map of pS63, a plasmid that was obtained by removing 3 regions from pIGPD7.
RB = right border region (about 220 bp, covering the real right border).
ORI = origin of replication of the plasmid.
Bin19 = right border sequence (25 bp,
GGTTTACCCGCCAATATATCCTGTC
Bin19 = left border sequence (24 bp,

### ATTTACAATTGAATATATCCTGCC

The decrease in size of the cassette works to improve transformation efficiency. Additionally the use of one or both T DNA borders can be used. Preferably one is used but both can be employed. We most frequently employ the right border sequence of T DNA (about 25 base pairs) though the left appears equally as useful. Thus forming genetically modified plant or plant cell, having in the cassette of the nucleotide sequence one or more T DNA border sequences which is not necessarily place in any specific location though the ends are often employed for these border regions.

In Fig. 1 the PatR1 and the PatR2 short fragments that include the entire pat gene and promoter are shown. The data is shown in Tables 2a and 2b. Tables 2c through 8 show the results of the Southern blot data in tabular form.

**Table 2c: Fragment r1/f2**

| No | Experiment | GUS staining | PAT probe | GUS probe | |
|---|---|---|---|---|---|
| 55 | f1 34 | - | >10 | >10 | blot 11b |
| 56 | | - | 1 | 1 | |
| 57 | | + | 4 | 4 | |
| 58 | f1 50 | + | >8 | <6 | |
| 59 | | - | 1 | ? | |
| 60 | | - | >3 | >2 | |
| 61 | f4 49 | - | 1 | 2 | |
| 62 | | - | >10 | >10 | |
| 63 | | - | 1 | - | |
| 64 | | - | 1 | - | |
| 65 | f4 71 | - | 2 | - | |
| 66 | | - | 9 | 7 | |

**Table 3: Fragment r1/f3**

| No | Experiment | GUS staining | PAT probe | GUS probe | |
|---|---|---|---|---|---|
| 67 | f5 | - | 1 | - | blot 11b |
| 68 | | - | 2 | 1 | |
| 69 | | - | 1 | - | |
| 70 | | - | 6 | 7 | same as 8? |
| 71 | | - | >5 | >5 | |
| 8 | | - | >10 | >10 | |
| 1 | | - | - | - | blot 12 |
| 2 | | - | 1 | - | |
| 3 | | + | 8 | 4 | |
| 4 | | - | - | - | |
| 5 | | - | - | - | |
| 6 | | - | - | - | |
| 7 | | - | - | - | |
| 8 | | - | 2 | 4 | |
| 9 | | - | 2 | 2 | |
| 10 | | - | 5 | 7 | |
| 11 | | - | >4 | >3 | |
| 12 | | - | >10 | >10 | |
| 78 | | ? | >6 | 3 | |

**Table 4: Fragment r4/f2**

| No | Experiment | GUS Staining | PAT probe | GUS probe | |
|---|---|---|---|---|---|
| 19 | f7 | + | >10 | >10 | blot 12a |
| 20 | | + | >10 | >10 | |
| 21 | | + | >10 | >10 | |
| 22 | | + | ? | ? | |
| 23 | | - | >10 | >10 | |
| 24 | | + | >10 | >10 | |
| 25 | | + | 6 | 4 | |
| 26 | | - | 3 | - | |
| 28 | | - | 6? | - | |
| 29 | | - | 1 | - | |
| 30 | | + | >10 | >10 | |
| 74 | f6 | ? | >10 | >10 | |
| 75 | | ? | 4 | 3 | |
| 76 | | ? | 2 | - | |
| 77 | | ? | - | - | DNA broken down |

**Table 5: Fragment r4/f3**

| No | Experiment | GUS Staining | PAT probe | GUS probe | |
|---|---|---|---|---|---|
| 72 | f6 | + | 2 | 1? | blot 11b |
| 73 | | + | >7 | >7 | |
| 13 | f7 | - | 1 | - | blot 12a |
| 14 | | - | 1 | - | |
| 15 | | - | >10 | >10 | |
| 16 | | - | 3? | 1 | |
| 17 | | - | 3 | 1 | |
| 18 | | - | 1 | - | |
| 127 | f6 | + | >10 | >10 | blot 14a |
| 128 | f8 | - | - | - | |
| 129 | | - | - | - | |
| 130 | | + | 1 | 2 | |
| 131 | | + | 6 | >10 | |
| 132 | | + | >10 | >10 | |

**Table 6: Fragment partr1/f2 (Number of bands on Southern blot)**

| No | Experiment | PAT probe | |
|---|---|---|---|
| 31 | f8 | >10 | blot 12a SST |
| 32 | | ? | |
| 100 | f9 | >10 | blot 14 EcoR1 |
| 101 | | >10 | |
| 102 | | >10 | |
| 103 | | 2 | |
| 104 | | 6 | |
| 105 | | - | |
| 106 | | 1 | |
| 107 | | 2 | |
| 108 | | 1 | |
| 109 | | ? | |
| 110 | | ? | |
| 111 | | >10 | |

**Table 7: Fragment partr2/f2**

| No | Experiment | PAT probe | |
|---|---|---|---|
| 33 | f9 | 3 | blot 12a SST |
| 34 | | 1 | |
| 112 | f9 | - | blot 14 EcoR1 |
| 113 | | 1 | |
| 114 | | 1 | |
| 115 | | 1 | |
| 116 | | 1 | |
| 117 | | - | |
| 118 | | 1 | |
| 119 | | - | |

**Table 8: Plants**

| No | Experiment | Fragment | GUS staining | PAT probe | GUS probe | |
|---|---|---|---|---|---|---|
| 1 | f6 | r4/f2 | - | - | - | blot 14 |
| 2 | f1 | r1/f2 | - | >10 | >10 | |
| 3 | f4 | r1/f2 | + | 6 | >10 | |
| 4 | f6 | r4/f2 | - | 2 | 3 | |

## Claims

1. A method for obtaining a genetically modified plant comprising the steps of:
(i) transforming, in the presence of PEG-medium, protoplasts, by introducing an hereditary material into the protoplasts of said plant in order to obtain transformants containing at least one copy of the hereditary material per protoplast,
(ii) selecting a stable transformant and
(iii) regenerating plant cells or a whole plant from said transformant,
**characterised in that** the hereditary material is a cassette nucleotide sequence to be inserted in the genome of said plant and being free or substantially free from other prokaryotic or plasmidic nucleotide sequences.

2. The method according to claim 1, **characterised in that** the cassette sequence comprises a genetic sequence selected from the group consisting of genetic sequences involved in the resistance to plant viruses (especially BNYVV), resistance to plant parasites (nematodes, fungi or insects), resistance to herbicides (preferably glyphosate, glufosinate, imathezethate and/or acetochloride resistance), insecticides or fungicides or involved in the improved metabolism of carbohydrates.

3. The method according to any one of the preceding claims, **characterised in that** the protoplasts are derived from the conversion of stomatal cells

4. The method according to claim 3, **characterised in that** the stomatal cells are guard cells.

5. The method according to any one of the preceding claims, **characterised in that** the cassette nucleotide sequence is an amplicon resulting from genetic amplification steps.

6. The method according to any one of the preceding claims 1, **characterised in that** the step of transforming protoplasts of the plant by introducing an hereditary material into the protoplasts is obtained with amplicons present at the concentration of higher than 10µg DNA /1 x 10⁶ protoplasts.

7. The method according to any one of the preceding claims, **characterised in that** it comprises the step of providing an intact tissue of said plant containing stomatal cells and converting said stomatal cells into protoplasts by enzymatic digestion of the cell walls, said step being performed before the step of transforming the protoplasts.

8. The method according to claim 7, **characterised in that** said plant tissue is senescent leaf tissue or leaf epidermis.

9. The method according to any one of the preceding claims, **characterised in that** the plant is a sugar beet (Beta vulgaris).

10. A genetically modified sugar beet (Beta vulgaris) plant or sugar beet plant cell, obtainable by a method according to any one of the claims 2 to 9.

11. The sugar beet plant or sugar beet plant cell according to claim 10, wherein the cassette nucleotide sequence to be inserted includes one or more T DNA border sequences.

## Patentansprüche

1. Verfahren für das Erhalten einer genetisch veränderten Pflanze, umfassend die Schritte der:
(i) Transformation von Protoplasten in Gegenwart von PEG-Medium, durch Einführung eines vererbbaren Materials in die Protoplasten von der Pflanze, um Transformanten zu erhalten, die mindestens eine Kopie von dem vererbbaren Material pro Protoplast enthalten,
(ii) Selektion eines stabilen Transformanten und
(iii) Neubildung von Pflanzenzellen oder einer ganzen Pflanze aus dem Transformanten,
**dadurch gekennzeichnet, dass** das vererbbare Material eine Nukleotidsequenz-Kassette ist, die in das Genom von der Pflanze eingefügt werden muss und frei ist oder im Wesentlichen frei ist von anderen prokaryotischen oder Plasmid-basierten Nukleotidsequenzen.

2. Verfahren nach Anspruch 1, **dadurch gekennzeichnet, dass** die Kassetten-Sequenz eine genetische Sequenz umfasst, die ausgewählt ist aus der Gruppe bestehend aus genetischen Sequenzen involviert in die Resistenz gegenüber Pflanzenviren (insbesondere BNYVV), Resistenz gegenüber Pflanzenparasiten (Nematoden, Pilze oder Insekten), Resistenz gegenüber Herbiziden (vorzugsweise Glyphosat, Glufosinat, Imathezethat und / oder Acetochlorid-Resistenz), Insektiziden oder Fungiziden, oder involviert in den verbesserten Metabolismus von Kohlehydraten.

3. Verfahren nach irgendeinem der vorhergehenden Ansprüche, **dadurch gekennzeichnet, dass** die Protoplasten aus der Umwandlung von stomatalen Zellen erhalten wurden.

4. Verfahren nach Anspruch 3, **dadurch gekennzeichnet, dass** die stomatalen Zellen Schließzellen sind.

5. Verfahren nach irgendeinem der vorhergehenden Ansprüche, **dadurch gekennzeichnet, dass** die Nukleotidsequenz-Kassette ein Amplikon ist, das aus genetischen Vervielfältigungsschritten resultiert.

6. Verfahren nach irgendeinem der vorhergehenden Ansprüche, **dadurch gekennzeichnet, dass** der Schritt der Transformation der Protoplasten der Pflanze, durch die Einführung eines vererbbaren Materials in die Protoplasten, mit Amplikons erhalten wird, die bei einer Konzentration von mehr als 10 µg DNA /1 x 10⁶ Protoplasten vorliegen.

7. Verfahren nach irgendeinem der vorhergehenden Ansprüche, **dadurch gekennzeichnet, dass** es den Schritt der Bereitstellung eines intakten Gewebes von der Pflanze umfasst, das stomatale Zellen enthält und der Umwandlung der stomatalen Zellen in Protoplasten durch enzymatische Verdauung der Zellwände, wobei der Schritt vor der Transformationsschritt der Protoplasten durchgeführt wird.

8. Verfahren nach Anspruch 7, **dadurch gekennzeichnet, dass** das Pflanzengewebe ruhendes Blattgewebe oder Blattepidermis ist.

9. Verfahren nach irgendeinem der vorhergehenden Ansprüche, **dadurch gekennzeichnet, dass** die Pflanze eine Zuckerrübe (Beta vulgaris) ist.

10. Genetisch veränderte Zuckerrüben-Pflanze (Beta vulgaris) oder Zuckerrüben-Pflanzenzelle, erhältlich durch ein Verfahren nach irgendeinem der Ansprüche 2 bis 9.

11. Zuckerrüben-Pflanze oder Zuckerrüben-Pflanzenzelle nach Anspruch 10, wobei die einzufügende Nukleotidsequenz-Kassette eine oder mehrere T-DNA Randsequenzen einschließt.

## Revendications

1. Procédé pour obtenir une plante génétiquement modifiée comprenant les étapes de :
(i) transformation de protoplastes, en présence d'un milieu de PEG, en introduisant une matière héréditaire dans les protoplastes de ladite plante pour obtenir des transformants contenant au moins une copie de la matière héréditaire par protoplaste,
(ii) sélection d'un transformant stable et
(iii) régénération de cellules végétales ou d'une plante entière à partir dudit transformant,
**caractérisé en ce que** la matière héréditaire est une séquence nucléotidique sous la forme d'une cassette à insérer au génome de ladite plante et exempt ou sensiblement exempt d'autres séquences nucléotidiques d'origines procaryote ou plasmidique.

2. Procédé selon la revendication 1, **caractérisé en ce que** la séquence sous forme de cassette comprend une séquence génétique choisie dans le groupe constitué des séquences génétiques impliquées dans la résistance à des virus de plantes (en particulier, le BNYVV), dans la résistance à des parasites de plantes (nématodes, champignons ou insectes), dans la résistance à des herbicides (de préférence, la résistance au glyphosate, au glufosinate, à l'imathézéthate et/ou au chlorure d'acétate), à des insecticides ou à des fongicides, ou qui sont impliquées dans le métabolisme renforcé des hydrates de carbone.

3. Procédé selon l'une quelconque des revendications précédentes, **caractérisé en ce que** les protoplastes dérivent de la conversion de cellules stomatiques.

4. Procédé selon la revendication 3, **caractérisé en ce que** les cellules stomatiques sont des cellules de garde.

5. Procédé selon l'une quelconque des revendications précédentes, **caractérisé en ce que** la séquence nucléotidique sous forme de cassette est un amplicon obtenu par des étapes d'amplification génétique.

6. Procédé selon l'une quelconque des revendications précédentes, **caractérisé en ce que** l'étape de transformation des protoplastes de la plante par introduction d'une matière héréditaire dans les protoplastes est obtenue avec des amplicons présents en concentration supérieure à 10 µg d'ADN/1 x 10⁶ protoplastes.

7. Procédé selon l'une quelconque des revendications précédentes, **caractérisé en ce qu'**il comprend l'étape consistant à fournir un tissu intact de ladite plante contenant des cellules stomatiques, et à convertir lesdites cellules stomatiques en protoplastes par digestion enzymatique des parois cellulaires, ladite étape étant réalisée avant l'étape de transformation des protoplastes.

8. Procédé selon la revendication 7, **caractérisé en ce que** ledit tissu végétal est un tissu de feuille ou un épiderme de feuille sénescent.

9. Procédé selon l'une quelconque des revendications précédentes, **caractérisé en ce que** la plante est une betterave sucrière (Beta vulgaris).

10. Plant de betterave sucrière génétiquement modifiée (Beta vulgaris) ou cellule végétale de plant de betterave sucrière, que l'on peut obtenir par un procédé selon l'une quelconque des revendications 2 à 9.

11. Plant de betterave sucrière ou cellule de plant de betterave sucrière selon la revendication 10, dans lequel la séquence nucléotidique sous forme de cassette à insérer comprend une ou plusieurs séquences de bordure d'ADN-T.
